# EUROPEAN PATENT SPECIFICATION

(11) **EP 1 429 725 B1**
(45) Date of publication and mention of the grant of the patent: **19.03.2008**
(21) Application number: 02779354.6
(22) Date of filing: 13.09.2002
(51) Int. Cl.: A61K 9/00, A61K 9/16, A61K 9/51, A61K 31/55, A61K 31/50, A61P 27/02

(54) **OPHTHALMIC DEPOT FORMULATIONS FOR PERIOCULAR OR SUBCONJUNCTIVAL ADMINISTRATION**
OPHTHALMISCHE DEPOT-FORMULIERUNGEN ZUR PERIOKKULAREN ODER SUBKONJUNKTIVALEN VERABREICHUNG
PREPARATIONS DE DEPOT OPHTALMIQUE A ADMINISTRATION PERIOCULAIRE OU SOUS-CONJONCTIVALE

(30) Priority: 14.09.2001 GB 0122318
(43) Date of publication of application: 23.06.2004
(73) Proprietor: Novartis AG, 4056 Basel (CH); Novartis Pharma GmbH, 1230 Vienna (AT)
(72) Inventor: AHLHEIM, Markus, 79219 Staufen (DE); AUSBORN, Michael, 79540 Lörrach (DE); BODMER, David, CH-5313 Klingnau (CH); SCHOCH, Christian, CH-4132 Muttenz (CH)
(74) Representative: de Weerd, Petrus G.W.
(86) International application number: PCT/EP2002/010314
(87) International publication number: WO 2003/024420

(56) References cited:
- EP-A- 0 322 319
- WO-A-01/74389
- WO-A-02/28366
- WO-A-98/27962
- DE-A- 3 722 837
- GB-A- 2 145 422
- US-A- 3 773 919
- US-A- 5 632 984
- US-A- 5 824 072

## Description

The present invention relates to the use of an ophthalmic formulation in the manufacture of a depot medicament for periocular administration for treatment of ocular diseases, in particular treatment of retinal and choroidal diseases, wherein said depot formulation is in accordance to the main claim.

Ocular diseases are difficult to treat as introduction of active agents into the eye and maintenance of therapeutically effective concentration thereof is difficult.

Oral administration of an active agent or parenteral administration of an active agent to a site other than the eye provides the active agent systemically. In order to achieve effective intraocular concentrations, systemic administration may necessitate administration of often unacceptably high levels of the active agent.

Injection of compositions comprising an active agent into the eye may be ineffective as the active agent may be washed out or is depleted from within the eye into the general circulation resulting in necessity for repeated administration, e.g. three injections in three to 42 days as described in US 5,632,984.

Introduction of slow release compositions, i.e. implants, into the eye, e.g. into an anterior segment or posterior segment of an eye as described in US 4,853,224, e.g. into the suprachoroidal space or pars plana of the eye as described in US 5,164,188, or e.g. into a site extrinsic to the vitreous comprising a suprachoroidal space, an avascular region of an eye, or a surgically-induced avascular region as described in US 5,824,072, by injection or surgical methods such as laser ablation, photocoagulation, cryotherapy, heat coagulation and the like is extremely painful and stressful for the patient. Implants may have to be removed when therapy is completed or no longer efficacious.

Applicants have found that ophthalmic depot formulations described herein below comprising an active agent may be administered, periocularly, e.g. retrobulbarly or sub-tenonly, or subconjunctivally.

Accordingly in one aspect, the present invention relates to the use of an ophthalmic formulation in the manufacture of a depot medicament for periocular, e.g. retrobulbar or subtenon, or subconjunctival administration in the treatment of an ocular disease, wherein said ophthalmic formulation comprises an active agent embedded in a biocompatible pharmacologically acceptable polymer, and wherein said polymer is a polylactide-co-glycolide ester of a polyol, and wherein said polyol contains at least 3 hydroxy groups and has a molecular weight of up to 20,000.

Ophthalmic depot formulations such as micro- or nanoparticle (hereinafter called microparticle) formulations, comprising an active agent e.g. embedded in a biocompatible pharmacologically acceptable polymer e.g. in an encapsulating polymeric matrix, have been found to be particularly suitable. The ophthalmic depot formulation may also comprise microparticles of essentially pure active agent, e.g. microparticles consisting of the active agent.

These microparticles have a high contact surface.

Typically an ophthalmic depot formulation as described herein comprises microparticles of essentially pure active agent.

The microparticles of essentially pure active agent, e.g. microparticles consisting of the active agent, may be in amorphous or crystalline form e.g. with a particle size of 1 to 200 microns.

The depot formulations, e.g. in particular microparticle formulations, being described herein are adapted to release all or substantially all the active material over an extended period of time, e.g. several weeks up to 6 months. The matrix, e.g. polymer matrix, if present, is adapted to degrade sufficiently to be transported from the site of administration within one to 6 months after release of all or substantially all the active agent.

The polymers used in this invention are linear polyesters, and branched chain polyesters. The linear polyesters may be prepared from the α-hydroxy carboxylic acids, e.g. lactic acid and glycolic acid, by the condensation of the lactone dimers, see e.g. US 3,773,919.

Linear polylactide-co-glycolides (PLG) which are preferably used conveniently have a molecular weight between 25,000 and 100,000 and a polydispersity M_{w}/Mₙ e.g. between 1.2 and 2.

The branched polyesters preferably used according to the invention may be prepared using polyhydroxy compounds e.g. polyol e.g. glucose or mannitol as the initiator. These esters of a polyol are known and described in GB 2,145,422 B. The polyol contains at least 3 hydroxy groups and has a molecular weight of up to 20,000, with at least 1, preferably at least 2, e.g. as a mean 3 of the hydroxy groups of the polyol being in the form of ester groups, which contain poly-lactide or co-poly-lactide chains. Typically 0.2% glucose is used to initiate polymerization. The branched polyesters (Glu-PLG) have a central glucose moiety having rays of linear polylactide chains, e.g. they have a star shaped structure. The preferred polyester chains in the linear and star polymer compounds preferably used according to the invention are copolymers of the alpha carboxylic acid moieties, lactic acid and glycolic acid, or of the lactone dimers. The molar ratios of lactide: glycolide is from about 75:25 to 25:75, e.g. 60:40 to 40:60, with from 55:45 to 45:55, e.g. 55:45 to 50:50 the most preferred.

The branched polyesters having a central glucose moiety having rays of linear polylactide chains (Glu-PLG) may be prepared by reacting a polyol with a lactide and preferably also a glycolide at an elevated temperature in the presence of a catalyst, which makes a ring opening polymerization feasible.

The branched polyesters having a central glucose moiety having rays of linear polylactide chains (Glu-PLG) preferably have an average molecular weight Mₙ in the range of from about 10,000 to 200,000, preferably 25,000 to 100,000, especially 35,000 to 60,000 and a polydispersity e.g. of from 1.7 to 3.0, e.g. 2.0 to 2.5. The intrinsic viscosities of star polymers of Mₙ 35,000 and Mₙ 60,000 are 0.36 respectively 0.51 dl/g in chloroform. A star polymer having a Mₙ 52,000 has a viscosity of 0.475 dl/g in chloroform.

The terms microsphere, microcapsule and microparticle are considered to be interchangeable with respect to the invention, and denote the encapsulation of the active agent by the polymer, preferably with the active agent distributed throughout the polymer, which is then a matrix for the active agent. In that case preferably the terms microsphere or more generally microparticle are used.

The microparticles, e.g. microspheres or microcapsules, may have a diameter from a few submicrons to a few millimeters, e.g. from about 0.01 microns to about 2 mm, e.g. from about 0.1 microns to about 500 microns. For pharmaceutical microparticles, diameters of at most about 250 microns, e.g. 10 to 200 microns, preferably 10 to 130 microns, more preferably 10 to 90 microns, even more preferably 10 to 60 microns, are strived for, e.g. in order to facilitate passage through an injection needle.

Typically, the active agent will be from about 1 to 80, more usually 10 to 75% by weight of the polymeric microparticles and from 1 to 20% by weight of the lipid microparticles.

In another aspect, the present invention describes a liquid formulation, comprising a pharmaceutical acceptable polymer as described in claim 1 and a dissolved or dispersed active agent. Upon injection, the polymer forms a depot at the injection site, e.g. by gelifying or precipitating.

The depot formulations, in particular microparticle formulations, described in the present invention are suitable for the incorporation of a large variety of water soluble or hydrophobic active agents.

Active agents of particular interest include
i) anti-glaucoma drugs, such as the beta-blockers, e.g. timolol maleate, betaxolol, carteolol and metipranolol; epinephrine and prodrugs; such as dipivefrin; carbonic anhydrase inhibitors; such as dorzolamide, brinzolamide, acetazolamide, dichlorphenamide and methazolamide; dopaminergics, prostaglandins, docosanoids, alpha2 agonists; angiotensin II antagonists; alpha1 antagonists; cannabinoids; endothelin antagonists;
ii) miotics, e.g. pilocarpine, acetylcholine chloride, isoflurophate, demecarium bromide, echothiophate iodide, phospholine iodide, carbachol, and physostigmine;
iii) drugs for treatment of macular degeneration, such as interferon, particularly α-interferon; transforming growth factor (TGF), e.g. TGF-β;
iv) anti-cataract and anti-proliferative diabetic retinopathy (PDR) drugs, such as aldose reductase inhibitors: e.g. tolrestat, or angiotensin-converting enzyme inhibitors, e.g. lisinopril, enalapril;
v) drugs for treatment of age-related exudative macular degeneration (AMD), e.g. ocular neovascular disease, such as staurosporines, phthalazine derivatives;
vi) anti-clotting agents, such as tissue plasminogen activator, urokinase, and streptokinase;
vii) drugs for treatment of ocular inflammatory diseases such as cortico-steroids; e.g. prednisolone, triamcinolone, dexamethasone, fluocinolone, cortisone, prednisolone and fluorometholone, non-steroidal anti-inflammatory drugs, such as ketorolac tromethamine, diclofenac sodium, indomethacin, flurbiprofen sodium, and suprofen;
viii) antibiotics, such as loridine (cephaloridine), chloramphenicol, clindamycin, amikacin, gentamicin, tobramycin, methicillin, lincomycin, oxacillin, penicillin, amphotericin B, polymyxin B, cephalosporin family, ampicillin, bacitracin, carbenicillin, cephalothin, colistin, erythromycin, streptomycin, neomycin, sulfacetamide, vancomycin, silver nitrate, sulfisoxazole diolamine, quinolones, and tetracycline;
ix) anti-fungal or anti-viral agents, such as miconazole, ketoconazole, idoxuridine, trifluridine, vidarabine (adenine arabinoside), acyclovir (acycloguanosine), gancyclovir, foscarnet sodium, cidofovir, valacyclovir, famciclovirtrisulfapyrimidine-2, nystatin, flucytosine, natamycin, aromatic diamidines e.g. dihydroxystilbamidine and piperazine derivatives, e.g. diethylcarbamaine;
x) cycloplegics and mydriatic agents, such as atropine, cyclopentolate, scopolamine, homatropine tropicamide and phenylephrine;
xi) drugs for the treatment of ocular neurodegenerative diseases such as isopropyl unoprostone, glutamate receptor antagonists, e.g. memantine, caspase inhibitors, calcium antagonists, sodium channel blockers, NOS-2 inhibitors or neurotrophic factors, e.g. glial derived neurotrophic factor (GDNF) or ciliary neurotrophic factor (CNTF);
xii) peptide drugs such as calcitonin, lypressin or a somatostatin or analogues thereof,
xiii) anti-VEGF drugs;
xiv) phosphodiesterase inhibitors;
xv) antisense drugs such as fomivirsen sodium;
xvi) immunosuppressive agents; such as azathioprine, cyclosporin A, methotrexate, colchicine;
xvii) drugs for the treatment of ocular angiogenesis such as angiostatic steroids, PKC inhibitors, VEGF antagonists, COX2 inhibitors, ACE inhibitors or angiotensin II antagonists;
xviii) free radical scavengers, e.g. alpha tocopherol, carotenoids, sulfhydryl-containing compounds.

Preferably, active agents are drugs for treatment of the orbit region and ocular appendages, and for treatment of retinal and choroidal diseases comprising age-related macular degeneration, diabetic retinopathy, glaucoma, inflammation, e.g. endophthalmitis, and bacterial, fungal or viral infections. Even more preferably, the active agent is a staurosporine of formula (I), as phthalazine of formula (II) or an ophthalmically acceptable salt thereof. Even more preferred are the staurosporine of formula (I) wherein R is benzoyl (hereinafter compound A), and the phthalazine of formula (II) wherein Z is 4-pyrididyl, X is imino, n is 0, and Y is 4-chlorophenyl (hereinafter compound B).

| | |
|---|---|
| 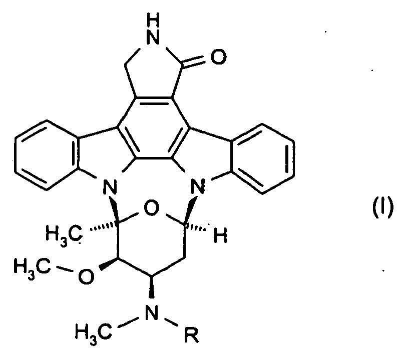 | 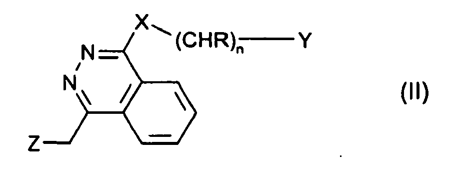 |
| | |
| wherein | wherein |
| R is benzoyl | n is 0 to 2, |
| | R is H or alkyl; |
| | X is imino, oxa, or thia; |
| | Y is aryl; and |
| | Z is unsubstituted or substituted pyridyl, or an N-oxide of the defined compound, wherein one or more N atoms carry an oxygen atom |

In another aspect, the present invention describes depot formulations and microparticles comprising a staurosporine of formula (I), a phthalazine of formula (II) or an ophthalmically acceptable salt thereof e.g. embedded in a biocompatible pharmacologically acceptable polymer of claim 1, e.g. for periocular, e.g. retrobulbar or sub-tenon, or subconjunctival administration.

The microparticles used in this invention may be prepared by any conventional technique, e.g. solvent evaporation, organic phase separation, spray drying, solvent extraction at low temperature or emulsion method, e.g. triple emulsion method. Using the phase separation or emulsion technique, the polymer is precipitated together with the drug, followed by hardening of the resulting product.

Also described is a process for the production of microparticles comprising the steps of
a) dissolving the polymer or lipid encapsulating agent and the active agent in an organic solvent, e.g. methylene chloride,
b) mixing the solution of a) with an aqueous solution of polyvinyl alcohol (e.g. 0.5%). e.g. using a static mixer
c) collecting the generated microparticles, e.g. by a sedimentation, filtration or using a cyclon,
d) optionally washing of microparticles e.g. in a buffered solution of e.g. pH 3.0 to 8.0 or distilled water, and
e) drying under vacuo e.g. at a temperature of 20°C to 40°C.

The microparticles described herein may be prepared by this process.

The microparticles and the depot formulations described herein are useful for treatment of the known ophthalmic indications of the particular active agent incorporated therein. The utility of the formulations of the present invention may be observed in standard animal trials and clinical trials.

There is further described a method for treating an ocular disease which comprises:
i) providing a depot formulation, e.g. a microparticle formulation, comprising an active agent e.g. embedded in a pharmacologically acceptable biocompatible polymer as described above, and
ii) administering said depot formulation, e.g. microparticle formulation, periocularly, e.g. retrobulbarly or sub-tenonly, or subconjunctivally.

This method permits diffusion of said active agent from said depot formulation, e.g. a microparticle formulation, to the site of said ocular disease, e.g. the choroid, optic nerve, retina or vitreous. Preferably, the active agent is maintained at an effective dosage for said ocular disease at the site of said ocular disease for an extended period of time, e.g. for several weeks up to 6 months.

The depot formulations, e.g. microparticle formulations described herein, may be administered, periocularly, e.g. retrobulbarly or sub-tenonly, or subjconjunctivally in a variety of ways including injection and trocar. Preferably, the active agent particles or the microparticles are suspended in a suitable liquid carrier.

The exact amount of active agent embedded in the polymer, i.e. the exact amount of depot formulation, e.g. microparticles formulation, to be administered depends on a number of factors, e.g. the condition to be treated, the desired duration of treatment, the rate of release of active agent and the degradability of the polymeric matrix. The amount of active agent required may be determined on the basis of known in vitro or in vivo techniques. Repeated administration of the depot formulation of the invention may be effected when the polymeric matrix has sufficiently degraded.

Large amounts of active agent, e.g. up to 300 mg of active agent, e.g. in form of a suspension, may be administered in a single administration, e.g. in one injection. Frequency of dosing is variably dependent upon the severity of the syndrome. For severe cases dosing may occur once a month. The frequency is reduced when signs of the disease state show improvement. At that time dosing may be as infrequent as one dose every four or five months.

Filling may be effected before or after sterilization of the depot formulation. Sterilization of the formulation of the present invention and the primary package can be effected, e.g. by gamma irradiation e.g. at an energy of 25kGy, without degradation of active agent and/or microparticles.

Following is a description by way of example only of depot formulations of this invention.

### Example 1 to 3: Preparations of microparticles

| | Ex. 1 | Ex. 2 | Ex. 3 |
|---|---|---|---|
| compound A | 0.10 g | 0.25 g | 0.50 g |
| Glu-PLG | 0.90 g | 0.75 g | 0.50 g |
| methylene chloride | 2.5 ml | 4.0 ml | 9.5 ml |
| 1.5% aq. polyvinyl alcohol | 500 ml | 600 ml | 900 ml |
| 0.5% aq. polyvinyl alcohol | 3 l | 3 l | 3 l |

Compound A and the polymer Glu-PLG are dissolved in the methylene chloride. The resulting solution is pumped through a static mixer together with a 1.5% solution of polyvinyl alcohol in water into a stirred solution of polyvinylalcohol in water (0.5%). The resulting suspension is heated to 42-48°C with stirring within 60 min and kept at that temperature for further 30 min before the mixture is cooled down to about 22°C within 50 min. The suspension is allowed to sediment for approximately 10 min. The aqueous solution of polyvinyl is reduced under vacuo. The microparticles are washed with water for approximately 5 min. After sedimentation for 10 min, the solution is removed and the microparticles are filtered through an Ultipor filter, washed with water and dried under vacuo.

## Claims

1. Use of an ophthalmic formulation in the manufacture of a depot medicament for a periocular administration in the treatment of an ocular disease, wherein said ophthalmic formulation comprises an active agent embedded in a biocompatible pharmacologically acceptable polymer, and wherein said polymer is a polylactide-co-glycolide ester of a polyol, and wherein said polyol contains at least 3 hydroxy groups and has a molecular weight of up to 20,000.

2. Use of claims 1, wherein said polymer is a 40/60 to 60/40 polylactide-co-glycolide ester of said polyol.

3. Use of claim 1, wherein said polyol is glucose or mannitol.

4. Use of claim 1, wherein said formulation comprises microparticles of pure active agent.

5. Use of claim 4 wherein the external surface of the microparticles is free of active agent.

6. Use of claim 1 wherein said formulation is liquid and wherein said active agent is dissolved or dispersed.

7. Use of claim 1 which formulation is administered retrobulbarly, sub-tenonly or suconjunctivally.

8. Use of claim 6, wherein said formulation forms a gel or a precipitate at the injection site of the eye.

9. Use of claim 4, wherein said microparticles have a diameter of from 10 to 200 microns, preferably 10 to 130 microns, more preferably 10 to 90 microns, even more preferably 10 to 60 microns.

10. Use of claim 1 wherein the active agent is a staurosporine of formula (I), a phthalazine of formula (II) or an ophthalmically acceptable salt thereof, wherein formulae (I) and (II) are as defined below.
| | |
|---|---|
| 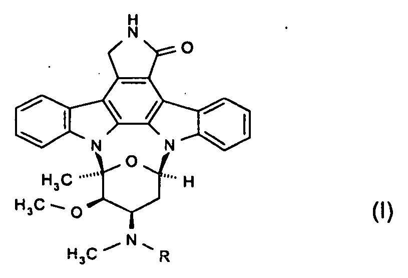 | 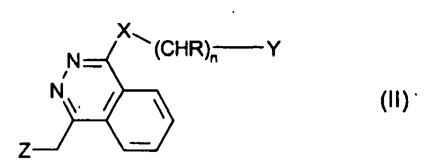 |
| | |
| wherein | wherein |
| R is benzoyl | n is 0 to 2, |
| | R is H or lalkyl; |
| | X is imino, oxa, or thia; |
| | Y is aryl; and |
| | Z is unsubstituted or substituted pyridyl, or an N-oxide of the defined compound, wherein one or more N atoms carry an oxygen atom |

## Patentansprüche

1. Verwendung einer ophthalmischen Formulierung zur Herstellung eines Depotarzneimittels für eine periokulare Verabreichung zur Behandlung einer Augenkrankheit, wobei die ophthalmische Formulierung einen Wirkstoff umfasst, der in einem biokompatiblen pharmakologisch akzeptablen Polymer eingebettet ist, worin dieses Polymer ein Polylactid-co-glycolidester eines Polyols ist und worin das Polyol wenigstens 3 Hydroxygruppen enthält und ein Molekulargewicht von bis zu 20000 hat.

2. Verwendung nach Anspruch 1, worin das Polymer ein 40/60 bis 60/40 Polylactid-co-glycolidester des Polyols ist.

3. Verwendung nach Anspruch 1, worin das Polyol Glucose oder Mannit ist.

4. Verwendung nach Anspruch 1, worin die Formulierung Mikropartikel von reinem Wirkstoff umfasst.

5. Verwendung nach Anspruch 4, worin die äußere Oberfläche der Mikropartikel frei von Wirkstoff ist.

6. Verwendung nach Anspruch 1, worin die Formulierung flüssig ist und worin der Wirkstoff gelöst oder dispergiert ist.

7. Verwendung nach Anspruch 1, worin die Formulierung retrobulbär, subtenonal oder subkonjunktival verabreicht wird.

8. Verwendung nach Anspruch 6, worin die Formulierung an der Injektionsstelle des Auges ein Gel oder ein Präzipitat bildet.

9. Verwendung nach Anspruch 4, worin die Mikropartikel einen Durchmesser von 10 bis 200 Mikron, vorzugsweise von 10 bis 130 Mikron, bevorzugter von 10 bis 90 Mikron und noch bevorzugter von 10 bis 60 Mikron haben.

10. Verwendung nach Anspruch 1, worin der Wirkstoff ein Stausporin der Formel (I), ein Phthalazin der Formel (II) oder ein ophthalmisch akzeptables Salz hiervon ist, worin die Formeln (I) und (II) wie folgt definiert sind:
| | |
|---|---|
| 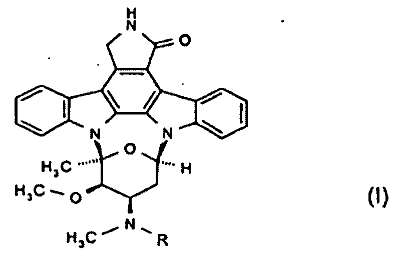 | 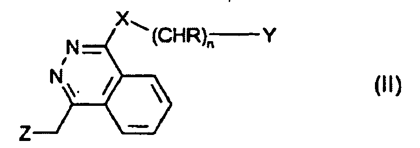 |
| worin | worin |
| R für Benzoyl steht | n für 0 bis 2 steht, |
| | R für H oder Alkyl steht, |
| | X für Imino, Oxa oder Thia steht, |
| | Y für Aryl steht, und |
| | Z für unsubstituiertes oder substituiertes Pyridyl steht, oder ein N-Oxid der definierten Verbindung worin ein oder mehr N-Atome ein Sauerstoffatom tragen. |

## Revendications

1. Utilisation d'une formulation ophtalmique dans la fabrication d'un médicament à effet retard destiné à une administration périoculaire dans le traitement d'une maladie oculaire, dans laquelle ladite formulation ophtalmique comprend un principe actif noyé dans un polymère biocompatible pharmacologiquement acceptable, et dans laquelle ledit polymère est un ester polylactide-co-glycolide d'un polyol, et dans laquelle ledit polyol contient au moins 3 groupes hydroxy et a une masse moléculaire allant jusqu'à 20 000.

2. Utilisation selon la revendication 1, dans laquelle ledit polymère est un ester polylactide-co-glycolide dudit polyol dans un rapport de 40/60 à 60/40.

3. Utilisation selon la revendication 1, dans laquelle ledit polyol est le glucose ou le mannitol.

4. Utilisation selon la revendication 1, dans laquelle ladite formulation comprend des microparticules de principe actif pur.

5. Utilisation selon la revendication 4, dans laquelle la surface externe des microparticules est exempte de principe actif.

6. Utilisation selon la revendication 1, dans laquelle ladite formulation est liquide et dans laquelle ledit principe actif est dissous ou dispersé.

7. Utilisation selon la revendication 1, la formulation étant administrée par voie rétrobulbaire, sous-ténonienne ou sous-conjonctivale.

8. Utilisation selon la revendication 6, dans laquelle ladite formulation forme un gel ou un précipité au site d'injection de l'oeil.

9. Utilisation selon la revendication 4, dans laquelle lesdites microparticules ont un diamètre de 10 à 200 microns, de préférence de 10 à 130 microns, plus préférablement de 10 à 90 microns, encore plus préférablement de 10 à 60 microns.

10. Utilisation selon la revendication 1, dans laquelle le principe actif est une staurosporine de formule (I), une phtalazine de formule (II) ou un sel ophtalmiquement acceptable de celles-ci, les formules (I) et (II) étant telles que définies ci-dessous.
| | |
|---|---|
| 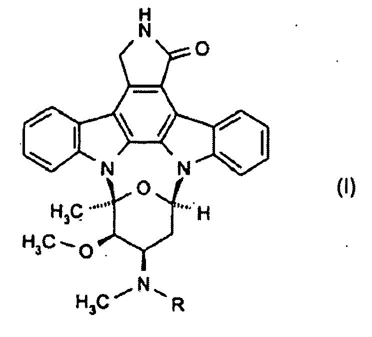 | 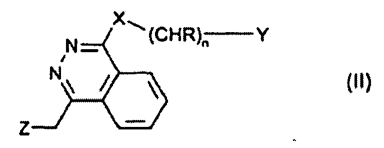 |
| dans laquelle | dans laquelle |
| R est un benzoyle | n vaut de 0 à 2, |
| | R est H ou un alkyle ; |
| | X est un imino, oxa ou thia ; |
| | Y est un aryle ; et |
| | Z est un pyridyle substitué ou non substitué, ou un N-oxyde du composé défini, où un ou plusieurs atomes N sont porteurs d'un atome d'oxygène |
